# EUROPEAN PATENT APPLICATION

(11) **EP 3 158 981 A1**
(43) Date of publication of application: **26.04.2017**
(21) Application number: 15812132.7
(22) Date of filing: 05.06.2015
(51) Int. Cl.: A61F 13/494

(54) **DISPOSABLE ABSORBENT ITEM WITH IMPROVED LEG ELASTICS**

(30) Priority: 23.06.2014 MX 2014007716
(71) Applicant: Grupo P.I. Mabe, S.A. de C.V., 72230 Puebla (MX)
(72) Inventor: SÁNCHEZ FERNÁNDEZ, Lucía Del Carmen, 72230 Puebla (MX); CANALES ESPINOSA DE LOS MONTEROS, Carlos, 72230 Puebla (MX)
(74) Representative: Gil-Vega, Victor
(86) International application number: PCT/IB2015/054251
(87) International publication number: WO 2015/198177

(57) **Abstract**

The present invention protects a disposable absorbent article which comprises basically an inner layer, an outer layer comprised by a laminate with two layers, one hydrophobic and one hydrophilic, an absorbent core placed between the inner layer and the outer layer and elastic areas at the waist and crotch parts formed by elastic threads or bands, such that the elastic crotch threads or bands are placed between the layers that form the laminate of the outer layer, such that the inner layer of the article and the inner layer of the laminate are placed between the elastic threads or bands and the skin of the user.

## Description

### Background

Disposable absorbent articles are articles which absorb and contain bodily exudates and which are discarded after use, including diapers and disposable panties for babies and adults, feminine hygiene products, panty protectors, etc. This type of articles has become an indispensable part of the daily routine for any family, which is why an infinity of technical designs have been developed to improve such products.

The use of threads or elastic bands in the crotch area of this type of articles, mainly disposable diapers and panties, is well known in the art, such that during use, these threads or elastic bands surround the thighs of the user, being adjusted in such a way that they form a seal with the skin to prevent fluids from leaking out of the article.

There are infinite proposals that aim to improve the functioning of the leg elastic areas of the disposable absorbent articles, such as patents US3860003, US3828784, US4050462, US4325372, US4397645, US4402688, US4425127, US4626305, US4657539, US4726807, US4854989, US4880420, US5275676, US5368584, US5649919, US7037301, US 8,690,851, among others.

In spite of these proposals, the problem persists in that the threads or elastic crotch bands area injure the skin of the user, leaving red marks and irritating the skin, one way to avoid this is to lower the tension in the threads or elastic bands, neverheless, this could diminish the functioning of the article by allowing fluid or semi-solid exudates to pass through this area.

A disposable absorbent article, such as a diaper or panty, is basically formed of an inner liquid-permeable layer, an outer liquid-impermeable layer, and an absorbent core positioned between both inner and outer layers; the threads or elastic leg bands are positioned between the inner and outer layers such that, during use, only the inner permeable layer remains positioned between the threads or elastic bands and the leg of the user. The inner layer of an article of this type is generally a non-woven fabric of low base weight, so it is not an adequate insulator to prevent the elastic band or threads from injuring the skin of the user. Moreover, it is desirable that the outer impermeable layer in this type of article has the appearance of fabric, for that reason, generally a laminate of two layers joined together is used as outer layer, one hydrophobic plastic film layer as the inner part of the laminate, and one non-woven fabric layer as the outer part, in order to obtain the fabric appearance. Thus, the present invention proposes that the elastic band or threads that form the adjustment area in the crotch of the article are provided between the two layers that form the laminate of the outer layer, in this way between the elastic band or threads and the skin there is a double layer of material that helps to smooth this area and isolate these elastic threads or band, protecting the skin of the user.

Moreover, the placement of the elastic band or threads between the layers of the outer layer laminate renders the possibility to broaden the width of the elastic waistband area of the article, since with the traditional placement between the inner and outer layers it is necessary to create a channel without adhesive on the front and back parts of the article, which extends upwards and downwards until the transverse edges, such that the elastic area of the waistband is limited to the distance between these channels. The above will be further explained bellow.

### Objectives of the invention

The objective of the present invention is to achieve an adequate adjustment of a disposable absorbent article to the skin of the user in the crotch elastic area, forming an efficient seal that does not injure the skin of the user.

Another objective of the invention is that this solution is viable, of easy application and does not increase the cost of the product.

An additional objective of the invention is that it renders the possibility of broadening of the width of the elastic waistband area, either on the front part and/or in the back part of the article.

### Brief Description of the Drawings

Figure 1 shows a diaper of the prior art.
Figure 2 shows the diaper of the invention
Figure 3 is a cross-section along line A-A' of Figure 2.

### Detailed Description of the invention

A disposable absorbent article is an article that absorbs and contains bodily exudates, and which is disposed after use. The present description will be provided based on a disposable baby diaper, in the understanding that it may also be applied to other type of disposable absorbent article, such as disposable diapers and panties for incontinent adults, training pants, feminine sanitary napkins, and incontinence pads.

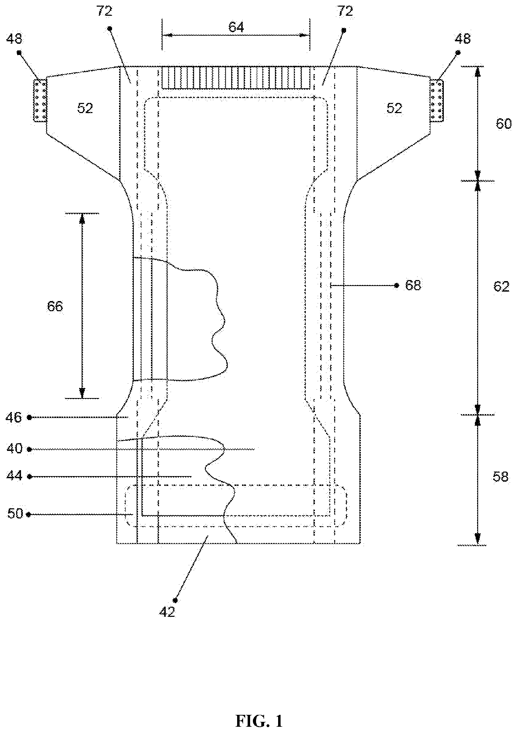

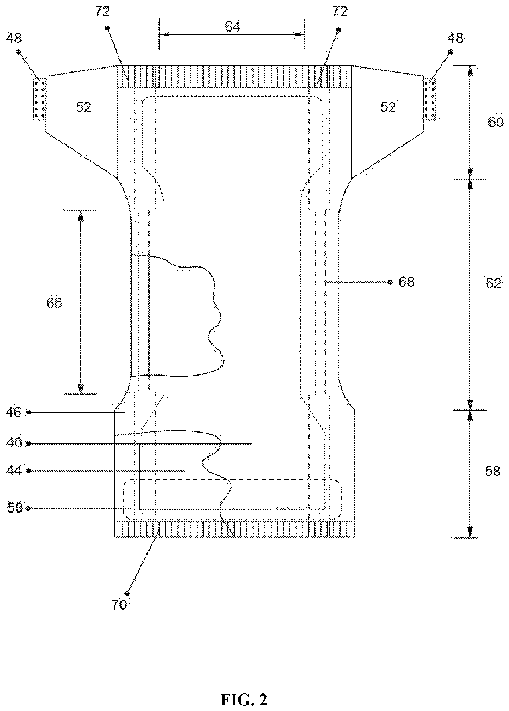

Figures 1 and 2 show a disposable diaper comprised by an inner layer (40) that is liquid permeable, an outer layer (42) that is liquid impermeable, an absorbent core (44) placed between both inner (40) and outer layers (42), a fastening system comprised by a pair of tape flaps (48) and a front band or tape (50); the diaper further has two elastic areas, one in the waist (64) and another at both longitudinal sides, in the crotch of the article, i.e., an elastic crotch area (66). The inner (40) and outer layers (42) abut one with the other and extend beyond the margins of the absorbent core (44), forming the contour of the diaper (46).

The absorbent articles such as disposable diapers have a front area (58), a back area (60) and a crotch area (62); the front area (58) is the one that will be positioned at the front waist and hip area of the user during use, the back area (60) will be positioned at the back waist and hip areas of same, while the crotch area (62) is the one that will be positioned between the legs and wrapping the thighs of the user during use.

As inner layer (40) is generally used a non-woven fabric of low base weight, between 6 and 20 g/m²; it may be hydrophilic or it may have a hydrophilic area in the central part and two hydrophobic areas on each side thereof. The non-woven fabric used as inner layer (40) may have any composition (polyethylene, polypropylene, polyester, cotton, cellulose, rayon, polylactic acid, etc.) and may be comprised by single, bi-, tri-components fibers, or mixtures thereof. Likewise, the fibers that comprise the fabric may be bonded using any process known in the field, such as pressure and heat bond using a calender, resin bonding, hot air bonding, etc., and may be comprised by one or several layers attached to each other.

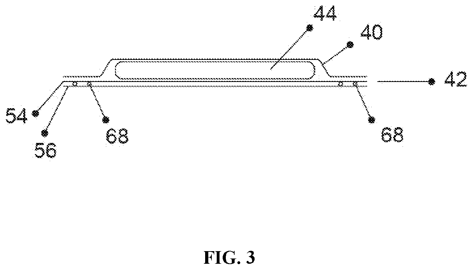

It is sought that this type of article is as similar as possible to underwear in terms of softness and appearance, thus, generally, a laminate formed by two layers, one inner (54) and one outer (56), is used as outer layer (42) as may be seen in Figure 3, that is a cross-section along the line A-A' shown in Figure 2. The inner layer (54) of the laminate is hydrophobic and the outer layer (56) may be hydrophilic or hydrophobic. Generally, a low-density polyethylene is used as inner layer (54); a non-woven hydrophobic fabric may also be used. A non-woven fabric is used as outer layer (56). The inner layer (54) of the laminate shall be positioned towards the inside of the article and shall prevent the pass of bodily fluids. The non-woven fabric of the outer layer (56) shall give the article a fabric-like appearance; this non-woven fabric gives the article the look and outer appearance, may be embossed or printed with several graphics to make the article attractive; any type of non-woven fabric such as those described for the inner layer (40) may be used provided that it has the suitable mechanical characteristics for laminating with the hydrophobic layer, as well as the required softness, flexibility and appearance. It may be hydrophilic or hydrophobic.

The absorbent core (44) of the article of the invention may be made of absorbent material fibers, such as defibrated cellulose, as well as other types of natural or synthetic absorbent fibers such as polyester, polypropylene, rayon, etc. The absorbent material fibers may be mixed with natural or synthetic superabsorbent material such as polyacrylates, starches, alginates, chitosan, etc. Figure 1 shows an "I" shaped absorbent core (44); however, same may have other shapes such as rectangular, hourglass, "T"-shaped, or any other shape that is deemed adequate.

The fastening system of the article is comprised by a pair of tape flaps (48) and a front band or tape (50). The tape flaps (48) are generally attached to the back waist part of the article, or, as shown in Figure 2, to a pair of ears (52) that may either be rigid or elastic and which, in turn, are attached to the back area (60) of the article; the tape flaps (48) may have elastic zones and rigid zones, or be completely rigid or completely elastic, may be adhesive or mechanical; the front band or tape (50) is positioned over the outer layer (42) at the front waist part of the article, and shall be selected from a suitable material so that the tape flaps (48) may be removably attached thereto, such that they may be removed and reattached thereto as much as necessary to adjust the article when placing it on the user, and may be removed again without scraping or damaging the article when it is removed from the user.

The elastic crotch areas (66) are formed by including elastic crotch threads or bands (68) in the crotch area (62) of the diaper. In the prior art articles, these elastic threads or bands (68) are tensioned and placed between the inner layer (40) and the outer layer (42), as shown in Figure 1. Thus, between the skin of the user and the elastic threads or bands (68), only the non-woven fabric layer from which the inner layer (40) of the article is made is interposed. As already mentioned, it is convenient for this non-woven fabric to have a low base weight between 6 and 20 gr/m². The use of a non-woven fabric of low base weight interposed between the elastic band or threads (68) and the skin of the user is not sufficient to prevent this elastic threads or bands (68) from injuring the skin, for which reason, according to the teachings of the present invention, the elastic crotch threads or bands are positioned between the layers that comprise the laminate of the outer layer (42) of the article as illustrated in Figures 2 and 3, in such a way that between the skin of the user and the elastic band or threads, the inner layer (54) of the laminate (42) and the non-woven fabric of the inner layer of the article (40) are interposed, thereby isolating the elastic band or threads (68) in order to protect the skin of the user.

On the other hand, the disposable absorbent articles are manufactured in continuous high-speed production lines, for placing the elastic crotch threads or bands, these are stretched until the desired tension, and are attached between the desired layers of the article while same is moving forward in the manufacturing line. In order to achieve this attachment, generally an adhesive is applied to the area in which the elastic bands or threads are desired to be active; the adhesive may be applied to any of the layers between which the elastic bands or threads are to be placed, or both; in this way, the elastic band or threads (68) are bonded with tension to the desired substrate in the crotch area (62). Since the adhesive must only be applied in the crotch area, an intermittent system is used that only applies adhesive in this area and do not apply it in front and back areas, in this way, a channel without adhesive (72) is created in the areas above and bellow of the active area of the elastics (74), i.e., this channel without adhesive extends from the area in which the adhesive is applied, to the transversal front and back edges of the article.

When the leg elastics are placed between the inner layer (40) and the outer layer (42), the elastic waist area (48) is limited to the width between these channels without adhesive (74), as may be seen in Figure 1 (prior art), because if the elastic waist threads or bands (46) are placed over the channels (74) it would be necessary to apply adhesive in this area, and the elastic crotch threads or bands (68) will be fixed with tension in the waist area, deforming the diaper.

When placing the elastic leg threads or bands (68) according to the teachings of the present invention, between the layers (54 and 56) that form the laminate of the outer layer, the channel without adhesive is formed between these layers and the elastic waist area (64) may be broadened up to the total width of the back waist part of the article, as shown in Figure 2, which illustrates the diaper of the invention, since adhesive used for attach the waist elastic to the diaper, is placed between the permeable inner layer (40) and the inner part of the impermeable layer (54) of the laminate that forms the outer layer (42) of the article, and thus the adhesive does not contact the channel without adhesive (72) which, in this case, is created between the inner layer (54) and the outer layer (56) of this laminate.

In the disposable absorbent articles industry, the laminate used as outer layer (42) may be acquired as such (already laminated) and be introduced into the production line of the disposable absorbent article as a single material or it may be formed as an additional process within the manufacturing line of the article. In the latter case, the production line has an additional module for forming the laminate; the non-woven fabric (56) (outer layer of the laminate) and the hydrophobic layer (54) (inner layer of the laminate) are fed to this module and are attached as a further step in the manufacturing process of the article, this is known as in-line lamination. The attaching means for bond the layers that form the laminate may be made by heat, adhesive, ultrasound bonding, or any other type of bonding known in the art. For the purposes of the present invention, the laminate that forms the outer layer (42) may be produced in-line, such that it is possible to introduce the elastic crotch threads or bands (68) between the layers that form the laminate.

On the other hand, when introducing the elastic threads or bands that comprise the elastic crotch areas (66) between the inner and outer layers of the outer layer laminate (42), it is possible to increase the elongation percentage in these areas, achieving an elongation of up to 400% without injuring the skin of the user.

While the invention has been described according to the currently preferred embodiments, it is evident that those skilled in the art could conceive changes or modifications, in the understanding that such changes or modifications are within the scope and spirit of the invention as defined in the appended claims.

## Claims

1. A disposable absorbent article that comprises basically an inner layer, an outer layer comprised by a laminate with two layers, one hydrophobic and one hydrophilic, an absorbent core placed between the inner layer and the outer layer and elastic areas at the waist and crotch parts formed by elastic threads or bands, wherein the elastic crotch threads and bands are placed between the layers that form the outer layer laminate, such that the inner layer of the article and the inner layer of the laminate are placed between the elastic threads or bands and the skin of the user.

2. A disposable absorbent article as described in claim 1, wherein the inner layer of the laminate is hydrophobic.

3. A disposable absorbent article as described in claim 2, wherein the inner layer of the laminate is a low-density polyethylene.

4. A disposable absorbent article as described in claim 2, wherein the inner layer of the laminate is a non-woven hydrophobic fabric.

5. A disposable absorbent article as described in claim 2, wherein the outer layer of the laminate is a non-woven fabric.

6. A disposable absorbent article as described in claim 1, wherein the elastic threads or bands are stretched to at least 200% before being attached between the inner and
outer layers of the laminate.

7. A disposable absorbent article as described in any of the preceding claims, wherein the elastic waist threads or bands are placed between the inner and outer layers of the article and have a width greater than the distance between the elastic crotch areas.
